(19) Europäisches Patentamt

European Patent Office

Office européen des brevets

(11) EP 0 555 634 B1

(12) EUROPÄISCHE PATENTSCHRIFT

(45) Veröffentlichungstag und Bekanntmachung des Hinweises auf die Patenterteilung:
**18.06.2003 Patentblatt 2003/25**

(51) Int Cl.[7]: **A61K 7/32**, A01N 31/02

(21) Anmeldenummer: **93100184.6**

(22) Anmeldetag: **08.01.1993**

(54) **Desinfektionsmittel**

Disinfection compositions

Compositions desinfectantes

(84) Benannte Vertragsstaaten:
**AT BE CH DE ES FR GB LI NL SE**

(30) Priorität: **10.01.1992 DE 4200499**

(43) Veröffentlichungstag der Anmeldung:
**18.08.1993 Patentblatt 1993/33**

(73) Patentinhaber: **Bode Chemie GmbH & Co.**
**22525 Hamburg (DE)**

(72) Erfinder:
• **Widulle,Herbert Dr.**
**W-2000 Hamburg 53 (DE)**
• **Pietsch,Hanns Dr.**
**W-2000 Hamburg 13 (DE)**
• **Möllers,Ulrich Dr.**
**W-2000 Hamburg 65 (DE)**
• **Ostermeyer,Christiane**
**W-2000 Hamburg 56 (DE)**

(74) Vertreter: **Dinné, Erlend**
**Beiersdorf AG**
**Unnastrasse 48**
**20245 Hamburg (DE)**

(56) Entgegenhaltungen:
DE-A- 4 004 014          FR-A- 2 159 383
GB-A- 1 024 501          GB-A- 2 001 649

**Beschreibung**

Die Erfindung betrifft alkoholische Desinfektionsmittel für die Hände und die Haut.

Für die Händedesinfektion und die Hautdesinfektion werden alkoholische Produkte eingesetzt, die ca. 60% bis 80% Alkohol enthalten. Der Alkohol ist das mikrobizide Agens. Die meisten dieser Desinfektionsmittel enthalten rückfettende Inhaltsstoffe für die Hautpflege, um einer Austrocknung der Haut durch die alkoholischen Wirkstoffe vorzubeugen. Trotzdem kann es insbesondere bei dem langen Tragen von undurchlässigen Handschuhen, wie es bei dem OP-Personal in Krankenhäusern bei der Arbeit üblich ist, zu Hautschäden kommen. Die Haut schwitzt und die Schweißflüssigkeit verbleibt auf der Haut und läßt diese quellen. Die oberen Hautschichten können auf diese Art vollständig zerstört werden, so daß ein weiteres Arbeiten unter solchen Bedingungen unmöglich wird.

Aus der Kosmetik ist ferner bekannt, daß Aluminiumsalze wie Aluminiumchlorid oder Aluminiumchlorhydrat schweißhemmend wirken. Manche dieser Mittel enthalten bis zu 20 Volumenprozent Alkohol. Eine desinfizierende Wirkung haben diese Formulierungen aber nicht. Ein Nachteil dieser Formulierungen ist auch, daß sie die Haut wegen der hohen Aluminiumsalzkonzentrationen von bis zu 20% (als $Al_2O_3$ berechnet) reizen können.

Aus der britischen Schrift GB-A-2 001 649 sind alkoholhaltige Deodorantien mit einem Gehalt an bestimmten Aluminium-Verbindungen bekannt.

Weiterhin ist in GB-A-10 24 501 ein hexachlorophenhaltiges Antiperspirant beschrieben.

Schließlich sind aus FR-A-2 159 383 antimikrobielle Zubereitungen mit einem Gehalt an basischen Aluminium-Halogen-Verbindungen bekannt.

Aufgabe der Erfindung war es, die Nachteile des Standes der Technik zu vermeiden und Desinfektionsmittel zu schaffen, die insbesondere die geschilderten Nachteile der Hautquellung durch Schweiß oder der Hautreizung nicht mehr oder nur in sehr geringem Maß besitzen, wobei diese Nachteile auch dann vermieden werden sollen, wenn die Desinfektionsmittel unter Gummihandschuhen, Pflastern oder Inzisionsfolien verwendet werden. Aufgabe der Erfindung ist es auch, die Rückverkeimung der Hautoberfläche zu verlangsamen und dadurch die Wirksamkeit der Mittel entscheidend zu verstärken.

Diese Aufgabe wird gelöst durch ein Händedesinfektionsmittel oder Hautdesinfektionsmittel mit mindestens einem alkoholischen Wirkstoff, das dadurch gekennzeichnet ist, daß es mindestens einen schweißvermindernden oder schweißhemmenden Stoff enthält.

Gegenstand der Erfindung ist daher die Verwendung mindestens eines schweißvermindernden oder schweißhemmenden Stoffes zur Herstellung eines Händedesinfektionsmittels oder Hautdesinfektionsmittels mit mindestens einem alkoholischen Wirkstoff, ausgenommen Aluminiumalkoholate und schwefelhaltige Aluminiumsalze.

Bevorzugte schweißvermindernde oder schweißhemmende Stoffe sind Aluminiumsalze, Aluminiumzirkoniumsalze, Zinksalze oder organische Verbindungen dieser Art und deren Gemische. Besonders bevorzugt werden

Aluminiumchlorid

Aluminium-hydroxychloride

Aluminium-zirkoniumchlorid

Aluminium-Zirkoniumchlorohydrate

Aluminium-Zirkoniumsulfat

Aluminium-nitrat

Aluminium-lactat

Aluminium-allantoinat

Zinkchlorid

Aluminiumsulfat

Aluminiumacetat

oder Zinksulfat.

Gemische können beispielsweise aus zwei oder drei Komponenten bestehen, vorzugsweise aber aus zwei Komponenten.

Bevorzugt sind Gemische von Aluminiumchlorid und Aluminiumhydroxychlorid, wobei das Mischungsverhältnis von reinem Aluminiumchlorid bis zum reinen Aluminiumhydroxychlorid variieren kann.

Besonders bevorzugte Gemische sind' Aluminiumchlorid/Aluminiumhydroxychlorid in den folgenden Gewichts-Verhältnissen 19:1, 8:14, 5:23 oder 1:19.

Vorzugsweise sind die schweißverhindernden oder schweißhemmenden Stoffe in Mengen von 0,001 bis 3 Gewichtsprozent, insbesondere 0,01 bis 3 Gewichtsprozent, besonders bevorzugt 0,02 bis 1,5 Gewichtsprozent und ganz besonders bevorzugt 0,04 bis 1 Gewichtsprozent enthalten, jeweils bezogen auf das gesamte Mittel.

Für die Metallverbindungen beziehen sich diese Gewichtsmengen auf die Metalloxide, insbesondere $Al_2O_3$, ZnO, $ZrO_2$ oder die Mischoxide.

Geeignete alkoholische Wirkstoffe sind z.B. monofunktionale Alkanole mit 1 bis 4 Kohlenstoffatomen.

Bevorzugte alkoholische Wirkstoffe sind Isopropanol, n-Propanol oder Ethanol oder Methanol und deren Ge-

EP 0 555 634 B1

mische. Diese Alkohole sind vorzugsweise in Mengen von 30 bis 95 Gewichtsprozent, insbesondere 50 bis 90 Gewichtsprozent, besonders bevorzugt 60 bis 80 Gewichtsprozent enthalten, jesweils bezogen auf das gesamte Mittel.

Bevorzugte Gemische der alkoholischen Wirkstoffe sind Ethanol/n-Propanol, Ethanol/iso-Propanol und iso-Propanol/n-Propanol, wobei für solche Gemische folgende Gewichtsprozente bevorzugt sind: der Anteil des Alkohols mit einem höheren Siedepunkt beträgt 10 bis 50 Gewichtsprozent und der Anteil des Alkohols mit niedrigerem Siedepunkt beträgt 60 bis 20 Gewichtsprozent, jeweils bezogen auf das gesamte Mittel.

Bevorzugt werden Desinfektionsmittel mit hohem Alkoholgehalt und zugleich niedrigem Gehalt an dem schweißvermindernden oder schweißhemmenden Stoff. Insbesondere beträgt der Alkoholgehalt über 60 Gew.-% z.B. 60 bis 95 Gew.-%, bevorzugt 60 bis 80 Gew.-% und der Gehalt an schweißverminderndem oder schweißhemmendem Stoff beträgt dabei bevorzugt 0,02 bis 1,5 Gew.-% und besonders bevorzugt 0,04 bis 1 Gew.-%, jeweils bezogen auf das gesamte Mittel.

Bevorzugt wird ein Mittel, das als schweißvermindernden oder schweißhemmenden Stoff ein Aluminiumsalz, Aluminiumzirkoniumsalz oder Zinksalz oder Gemische davon enthält sowie ein Mittel, das als schweißvermindernden oder schweißhemmenden Stoff Aluminiumchlorid oder Aluminiumhydroxychlorid oder Gemische davon enthält sowie ein Mittel, das eine Mischung aus Aluminiumchlorid und Aluminiumhydroxychlorid enthält wobei die Menge 0,04 bis 1 Gewichtsprozent berechnet als $Al_2O_3$ beträgt, jeweils bezogen auf das gesamte Mittel.

Bevorzugt wird auch ein Mittel, das als alkoholische Wirkstoffe n-Propanol und Isopropanol enthält sowie ein Mittel, das 30 Gewichtsprozent n-Propanol und 45 Gewichtsprozent Isopropanol enthält, sowie ein Mittel, das 30 bis 95 Gewichtsprozent alkoholische Wirkstoffe und 0,001 bis 3 Gewichtsprozent eines schweißvermindernden oder schweißhemmenden Stoffes enthält, jeweils bezogen auf das gesamte Mittel.

Bevorzugt wird auch ein Mittel, das 30 Gewichtsprozent n-Propanol, 45 Gewichtsprozent Isopropanol und eine Mischung aus Aluminiumchlorid und Aluminiumhydroxychlorid enthält, wobei die Menge 0,04 bis 1 Gewichtsprozent berechnet als $Al_2O_3$ beträgt, jeweils bezogen auf das gesamte Mittel.

Weiterhin können die Desinfektionsmittel auch Remanenzwirkstoffe enthalten, beispielsweise Chlorhexidindigluconat, Mecetroniumetilsulfat, Jod, Benzalkoniumchlorid, Wasserstoffperoxid oder andere Peroxide, Peressigsäure oder andere Persäuren oder Phenole wie Tetrabrommethylphenol oder o-Phenylphenol oder Gemische davon. Bevorzugte Mengen sind 0,025 bis 5 Gewichtsprozent.

Weiterhin kann in den Desinfektionsmitteln Wasser enthalten sein, vorzugsweise in Mengen von 10 bis 60 Gewichtsprozent, besonders bevorzugt in Mengen von 0 bis 30 Gew.-%, jeweils bezogen auf das gesamte Mittel. Bevorzugt sind entmineralisiertes Wasser, insbesondere aber aqua purificata (DAB 9).

Weiterhin können die Desinfektionsmittel einen oder mehrere Hautpflegestoffe, insbesondere Rückfetter enthalten. Bevorzugt sind Paraffinium subliquidum, Isostearylisostearat, Glycerin, Allantoin, Isopropylmyristat, Isopropylpalmitat oder Milchsäure oder Gemische davon.

Diese Hautpflegestoffe können z.B. in Mengen von 0,1 - 3 Gewichtsprozent enthalten sein.

Auch der Zusatz von Netzmitteln oder Tensiden, wie z.B. Ethercarbonsäuren, Fettalkoholethoxylaten, an sich bekannten Amphotensiden z.B. in Mengen von 0,1 bis 3,0 Gew.-% ist geeignet.

Außerdem können in dem Mittel weitere Hilfsstoffe, z.B. Farbstoffe, Färbemittel für die Haut oder Parfüm enthalten sein.

Bevorzugte erfindungsgemäße Desinfektionsmittel haben folgende Zusammensetzung:

|  | Gewichtsprozent |
|---|---|
| alkoholische Wirkstoffe | 30 bis 95 |
| schweißvermindernder oder schweißhemmender Stoff | 0,001 bis 3 |
| Wasser | 2 bis 60 |
| gegebenenfalls Rückfetter (insbesondere für Händedesinfektionsmittel) | 0,1 bis 3 |

Auch können Remanenzwirkstoffe und Hilfsstoffe, insbesondere in den angegebenen Mengen, zugefügt werden.

Die Herstellung der erfindungsgemäßen Mittel erfolgt in an sich bekannter Weise. In der gewünschten Wassermenge und/oder Alkoholmenge werden die schweißvermindernden oder schweißhemmenden Stoffe gelöst. Dann werden die Alkohole und gegebenenfalls die weiteren Inhaltsstoffe unter Rühren zugegeben. Dabei kann es hilfreich sein, die Temperatur bis auf etwa 25°C zu erhöhen.

Auch die Verwendung dieser Kombination von mindestens einem alkoholischen Wirkstoff und mindestens einem schweißvermindernden oder schweißhemmenden Stoff zur Herstellung eines Desinfektionsmittels zur Verringerung der Rückverkeimung, insbesondere bei occlusiven Bedingungen ist Gegenstand der Erfindung.

Tests ergaben, daß die Desinfektionsmittel überraschenderweise eine wesentlich geringere Rückverkeimung bewirken, wie auch der Versuchsbericht zeigt. Ein solcher Wiederanstieg der Verkeimung tritt immer durch nachwandernde Keime auf und beeinträchtigt in hohem Maß die Wirkung eines Desinfektionsmittels. Die Rückverkeimung war bei offener Anwendung aber auch bei occlusiven Bedingungen unerwartet stark verringert.

Außerdem verringern schon geringe Zusätze des schweißverminderndem oder schweißhemmenden Stoffes überraschend das Schwitzen, besonders unter einer Abdeckung wie Handschuhen, Pflastern und Inzisionsfolien so gut, daß die Quellung der Haut und damit die Hautschädigung vermindert oder vermieden wird. Weiter bewirken diese geringen Zusätze auch keine Hautreizung.

Die Desinfektionsmittel können durch wiederholtes Einreiben in die Haut der Hände und Unterarme, durch Auftragen mit einem Tupfer auf die Körperhaut oder Aufsprühen angewendet werden.

Die Desinfektionsmittel sind zum Dauergebrauch geeignet.

Die Desinfektionsmittel eignen sich besonders für die folgenden Zwecke: präoperative Händedesinfektion des Personals, Händedesinfektion zur Unterbrechung von Infektionsketten, Hautdesinfektion vor Operationen, Punktionen, Injektionen oder sonstigen Wundsetzungen,

Dabei zeigen sich die Vorzüge des neuen Mittels bevorzugt dann, wenn die Haut nach der Desinfektion occlusiv bedeckt wird mit z.B. OP-Handschuhen, Pflastern oder Inzisionsfolien.

Gegenstand der Erfindung ist auch die Verwendung eines schweißvermindernden oder schweißhemmenden Stoffes in einem Händedesinfektionsmittel oder einem Hautdesinfektionsmittel sowie die Verwendung eines schweißvermindernden oder schweißhemmenden Stoffes in einem Händedesinfektionsmittel oder einem Hautdesinfektionsmittel zur Verringerung der Rückverkeimung sowie die Verwendung eines schweißvermindernden oder schweißhemmenden Stoffes in einem Händedesinfektionsmittel oder einem Hautdesinfektionsmittel zur Verringerung der Rückverkeimung bei occlusiven Bedingungen sowie die Verwendung des erfindungsgemäßen Mittels bei nachfolgenden occlusiven Bedingungen.

Alle Prozentangaben oder Teile beziehen sich, soweit nicht anders angegeben, auf das Gewicht, insbesondere auf das Gesamtgewicht der Zubereitungen.

Die folgenden Beispiele dienen zur Erläuterung der Erfindung.

Beispiel 1

Händedesinfektionsmittel und Hautdesinfektionsmittel

Aus den folgenden Bestandteilen wird ein Desinfektionsmittel hergestellt:

|  | Gewichtsprozent |
|---|---|
| Glycerin | 0,50 |
| Propanol-1 | 30,00 |
| Propanol-2 | 45,00 |
| Paraffinum subliquidum | 0,75 |
| Mecetroniumetilsulfat | 0,20 |
| Parfüm | 0,10 |
| Aluminiumchlorid | 0,54 |
| Dialuminiumpentahydroxychlorid mit 2,5 $H_2O$ (zusammen entsprechend 0,8 Gew.-% $Al_2O_3$) | 1,26 |
| Wasser | 21,65 |
|  | $\overline{100}$ |

Zur Herstellung werden die Aluminiumverbindungen und das Mecetroniumetilsulfat in Wasser gelöst, dann werden die übrigen Bestandteile unter Rühren zugegeben.

Beispiel 2

Hautdesinfektionsmittel

|  | Gewichtsprozent |
|---|---|
| 1-Propanol | 60,00 |
| Aluminiumchlorid-hexahydrat (entsprechend 0,213 Gew.-% $Al_2O_3$) | 1,00 |

(fortgesetzt)

|  | Gewichtsprozent |
|---|---|
| Wasser | 39,00 |
|  | $\overline{100}$ |

Die Herstellung erfolgt wie vorstehend angegeben.

Beispiel 3

Händedesinfektionsmittel

|  | Gewichtsprozent |
|---|---|
| Ethanol | 80,00 |
| Glycerin | 0,25 |
| Isostearylisostearat | 0,20 |
| Parfüm | 0,03 |
| Aluminiumchlorid-hexahydrat (entsprechend 0,053 Gew.-% $Al_2O_3$) | 0,25 |
| Wasser | 19,27 |
|  | $\overline{100}$ |

Die Herstellung erfolgt wie vorstehend angegeben.

A. Versuchsbericht

Nach den Regeln für chirurgische Händedesinfektion der DGHM (Deutsche Gesellschaft für Hygiene und Mikrobiologie) desinfizierten sich 20 Probanden mit dem erfindungsgemäßen Desinfektionsmittel gemäß Beispiel 1 (Gruppe (a)). Danach wurde auf einer Hand ein enganliegender Handschuh aus wasserundurchlässigem und luftundurchlässigem Material getragen.

Dieselben Probanden desinfizierten sich 2 Wochen später in gleicher Weise mit einem Desinfektionsmittel wie in Beispiel 1 angegeben, in dem aber die Aluminiumverbindungen nicht enthalten und durch Wasser ersetzt waren (Gruppe (b)).

In beiden Fällen (a) und (b) lagen die Dosierungen zwischen 4,5 ml und 9 ml pro Hand.

Von Gruppe (a) wurde die behandschuhte Hand durchweg besser beurteilt als von Gruppe (b), solange der Handschuh getragen wurde.

Fünf Personen der Gruppe (a) beurteilten das Hautgefühl unter dem Handschuh als angenehm trocken. Die Haut war nicht gequollen. Zehn Probanden (a) empfanden das Hautgefühl unter dem Handschuh als normal.

Die Probanden der Gruppe (b) berichteten überwiegend über ein unangenehmes, feuchtes Hautgefühl. Die Haut war bei der Mehrzahl der Probanden weiß und gequollen.

B. Mikrobiologischer Leistungstest zur Ermittlung der Rückverkeimung

Für die Probandengruppen (a) und (b) wurde nach 3 Stunden Handschuhbenutzung die Rückverkeimung nach dem Leistungstest der DGHM durchgeführt. Es ergab sich für die Gruppe (a), die das erfindungsgemäße Desinfektionsmittel des Beispiels 1 verwendet hatte, eine siebenmal geringere Verkeimung im Vergleich zu dem Ergebnis der Gruppe (b). Dieses Ergebnis ist statistisch signifikant und der Unterschied der Meßergebnisse ist größer als die Fehlerbreite der Versuchswerte.

Aus den Versuchsberichten A und B ist die Überlegenheit der erfindungsgemäßen Desinfektionsmittel ersichtlich. Hautquellung oder Hautschäden oder Hautreizungen werden vermieden und die Rückverkeimung wird entscheidend verzögert und verringert.

**Patentansprüche**

1. Verwendung mindestens eines schweißvermindemden oder schweißhemmenden Stoffes zur Herstellung eines Händedesinfektionsmittels oder Hautdesinfektionsmittels mit mindestens einem alkoholischen Wirkstoff, ausgenommen Aluminiumalkoholate und schwefelhaltige Aluminiumsalze.

2. Verwendung gemäß Anspruch 1, **dadurch gekennzeichnet, daß** als schweißvermindernder oder schweißhemmender Stoff ein Aluminiumsalz, Aluminiumzirkoniumsalz oder Zinksalz oder Gemische davon verwendet werden.

3. Verwendung gemäß Anspruch 1, **dadurch gekennzeichnet, daß** als schweißvermindernden oder schweißhemmender Stoff Aluminiumchlorid oder Aluminiumhydroxychlorid oder Gemische davon verwendet werden.

4. Verwendung gemäß Anspruch 3, **dadurch gekennzeichnet, daß** eine Mischung aus Aluminiumchlorid und Aluminiumhydroxychlorid verwendet wird und die Menge 0,04 bis 1 Gewichtsprozent berechnet als $Al_2O_3$ beträgt, jeweils bezogen auf das gesamte Mittel.

5. Verwendung gemäß Anspruch 1, **dadurch gekennzeichnet, daß** als alkoholischer Wirkstoff Isopropanol, n-Propanol oder Ethanol oder Gemische davon verwendet werden.

6. Verwendung gemäß Anspruch 1, **dadurch gekennzeichnet, daß** als alkoholische Wirkstoffe n-Propanol und Isopropanol verwendet werden.

7. Verwendung gemäß Anspruch 1, **dadurch gekennzeichnet, daß** 30 Gewichtsprozent n-Propanol und 45 Gewichtsprozent Isopropanol verwendet werden, jeweils bezogen auf das gesamte Mittel.

8. Verwendung gemäß Anspruch 1, **dadurch gekennzeichnet, daß** 30 bis 95 Gewichtsprozent alkoholische Wirkstoffe und 0,001 bis 3 Gewichtsprozent eines schweißvermindernden oder schweißhemmenden Stoffes verwendet werden, jeweils bezogen auf das gesamte Mittel.

9. Verwendung gemäß Anspruch 1, **dadurch gekennzeichnet, daß** ein oder mehrere Hautpflegestoffezusätzlich verwendet werden.

10. Verwendung gemäß Anspruch 1, **dadurch gekennzeichnet, daß** ein Mittel enthaltend

|  | Gewichtsprozent |
|---|---|
| Glycerin | 0,50 |
| Propanol-1 | 30,00 |
| Propanol-2 | 45,00 |
| Paraffinum subliquidum | 0,75 |
| Mecetroniumetilsulfat | 0,20 |
| Parfüm | 0,10 |
| Aluminiumchlorid | 0,54 |
| Dialuminiumpentahydroxychlorid mit 2,5 $H_2O$ (zusammen entsprechend 0,8 Gew.-% $Al_2O_3$) | 1,26 |
| Wasser | 21,65 |

hergestellt wird.

11. Verwendung gemäß Anspruch 1, **dadurch gekennzeichnet, daß** 30 Gewichtsprozent n-Propanol, 45 Gewichtsprozent Isopropanol und eine Mischung aus Aluminiumchlorid und Aluminiumhydroxychlorid verwendet werden, wobei die Menge 0,04 bis 1 Gewichtsprozent berechnet als $Al_2O_3$ beträgt.

12. Verwendung gemäß Anspruch 1, **dadurch gekennzeichnet, daß** ein Mittel enthaltend

|  | Gew.-% |
|---|---|
| Ethanol | 80,00 |
| Glycerin | 0,25 |
| Isostearylisostearat | 0,20 |
| Parfüm | 0,03 |
| Aluminiumchloridhexahydrat (entsprechend 0,053 Gew.-% $Al_2O_3$) | 0,25 |
| Wasser | 19,27 |

hergestellt wird.

13. Verwendung mindestens eines alkoholischen Wirkstoffes und mindestens eines schweißvermindernden oder schweißhemmenden Stoffes zur Herstellung eine Händedesinfektionsmittels oder eines Hautdesinfektionsmittels zur Verringerung der Rückverkeimung, insbesondere bei occlusiven Bedingungen.

**Claims**

1. Use of at least one perspiration-reducing or antiperspirant substance for the preparation of a hand disinfectant or skin disinfectant with at least one alcoholic active ingredient, with the exception of aluminium alkoxides and sulphur-containing aluminium salts.

2. Use according to Claim 1, **characterized in that** an aluminium salt, aluminium zirconium salt or zinc salt or mixtures thereof are used as the perspiration-reducing or antiperspirant substance.

3. Use according to Claim 1, **characterized in that** aluminium chloride or aluminium hydroxychloride or mixtures thereof are used as perspiration-reducing or antiperspirant substance.

4. Use according to Claim 3, **characterized in that** a mixture of aluminium chloride and aluminium hydroxychloride is used, and the amount is from 0.04 to 1% by weight, calculated as $Al_2O_3$, in each case based on the overall composition.

5. Use according to Claim 1, **characterized in that** isopropanol, n-propanol or ethanol or mixtures thereof are used, as alcoholic active ingredient.

6. Use according to Claim 1, **characterized in that** n-propanol and isopropanol are used as alcoholic active ingredients.

7. Use according to Claim 1, **characterized in that** 30% by weight of n-propanol and 45% by weight of isopropanol are used, in each case based on the overall composition.

8. Use according to Claim 1, **characterized in that** from 30 to 95% by weight of alcoholic active ingredients and from 0.001 to 3% by weight of a perspiration-reducing or antiperspirant substance are used, in each case based on the overall composition.

9. Use according to Claim 1, **characterized in that** one or more skincare substances are used.

10. Use according to Claim 1, **characterized in that** a composition comprising

|  | Per cent by weight |
|---|---|
| Glycerol | 0.50 |
| 1-Propanol | 30.00 |
| 2-Propanol | 45.00 |
| Paraffinum subliquidum | 0.75 |

(continued)

|  | Per cent by weight |
|---|---|
| Mecetronium ethylsulphate | 0.20 |
| Perfume | 0.10 |
| Aluminium chloride | 0.54 |
| Dialuminium pentahydroxychloride containing 2.5 $H_2O$ (together corresponding to 0.8% by weight of $Al_2O_3$) | 1.26 |
| Water | 21.65 |

is produced.

11. Use according to Claim 1, **characterized in that** 30% by weight of n-propanol, 45% by weight of isopropanol and a mixture of aluminium chloride and aluminium hydroxychloride are used , where the amount is from 0.04 to 1% by weight calculated as $Al_2O_3$.

12. Use according to Claim 1, **characterized in that** a composition comprising

|  | % by weight |
|---|---|
| Ethanol | 80.00 |
| Glycerol | 0.25 |
| Isostearyl isostearate | 0.20 |
| Perfume | 0.03 |
| Aluminium chloride hexahydrate (corresponding to 0.053% by weight of $Al_2O_3$) | 0.25 |
| Water | 19.27 |

is produced.

13. Use of at least one alcoholic active ingredient and at least one perspiration-reducing or antipersipirant substance for the preparation of a hand disinfectant or a skin disinfectant for reducing microbial regrowth, in particular in occlusive conditions.

## Revendications

1. Utilisation d'au moins une substance réduisant la transpiration ou anti-transpiration pour la préparation d'une composition désinfectante pour les mains ou d'une composition désinfectante pour la peau comprenant au moins un ingrédient actif alcoolique, à l'exception des alcoolates d'aluminium et des sels d'aluminium soufrés.

2. Utilisation selon la revendication 1, **caractérisée en ce que** l'on utilise, en tant que substance réduisant la transpiration ou anti-transpiration, un sel d'aluminium, un sel d'aluminium-zirconium ou un sel de zinc, ou leurs mélanges.

3. Utilisation selon la revendication 1, **caractérisée en ce que** l'on utilise, en tant que substance réduisant la transpiration ou anti-transpiration, du chlorure d'aluminium ou de l'hydroxychlorure d'aluminium, ou leurs mélanges.

4. Utilisation selon la revendication 3, **caractérisée en ce que** l'on utilise un mélange de chlorure d'aluminium et d'hydroxychlorure d'aluminium et la quantité est de 0,04 à 1 pour cent en poids, calculée en $Al_2O_3$, dans chaque cas par rapport à la composition totale.

5. Utilisation selon la revendication 1, **caractérisée en ce que** l'on utilise, en tant qu'ingrédient actif alcoolique, de l'isopropanol, du n-propanol ou de l'éthanol, ou leurs mélanges.

6. Utilisation selon la revendication 1, **caractérisée en ce que** l'on utilise, en tant qu'ingrédients actifs alcooliques, du n-propanol et de l'isopropanol.

**7.** Utilisation selon la revendication 1, **caractérisée en ce que** l'on utilise 30 pour cent en poids de n-propanol et 45 pour cent en poids d'isopropanol, dans chaque cas par rapport à la composition totale.

**8.** Utilisation selon la revendication 1, **caractérisée en ce que** l'on utilise de 30 à 95 pour cent en poids d'ingrédients actifs alcooliques et de 0,001 à 3 pour cent en poids d'une substance réduisant la transpiration ou anti-transpiration, dans chaque cas par rapport à la composition totale.

**9.** Utilisation selon la revendication 1, **caractérisée en ce que** l'on utilise en outre une ou plusieurs substances de soins de la peau.

**10.** Utilisation selon la revendication 1, **caractérisée en ce que** l'on prépare une composition comprenant

| | Pour cent en poids |
|---|---|
| Glycérine | 0,50 |
| 1-Propanol | 30,00 |
| 2-Propanol | 45,00 |
| Paraffinum subliquidum | 0,75 |
| Mécétronium éthylsulfate | 0,20 |
| Parfum | 0,10 |
| Chlorure d'aluminium | 0,54 |
| Pentahydroxychlorure de dialuminium renfermant | 1,26 |
| 2,5 $H_2O$ (correspondant conjointement à 0,8 % en poids de $Al_2O_3$) | |
| Eau | 21,65. |

**11.** Utilisation selon la revendication 1, **caractérisée en ce que** l'on utilise 30 pour cent en poids de n-propanol, 45 pour cent d'isopropanol et un mélange de chlorure d'aluminium et d'hydroxychlorure d'aluminium, la quantité étant de 0,04 à 1 pour cent en poids, calculée en $Al_2O_3$.

**12.** Utilisation selon la revendication 1, **caractérisée en ce que** l'on prépare une composition comprenant

| | Pour cent en poids |
|---|---|
| Ethanol | 80,00 |
| Glycérine | 0,25 |
| Isostéarate d'isostéaryle | 0,20 |
| Parfum | 0,03 |
| Hexahydrate de chlorure d'aluminium | 0,25 |
| (correspondant à 0,053 % en poids de $Al_2O_3$) | |
| Eau | 19,27. |

**13.** Utilisation d'au moins un ingrédient actif alcoolique et d'au moins une substance réduisant la transpiration ou anti-transpiration pour la préparation d'une composition désinfectante pour les mains ou d'une composition désinfectante pour la peau en vue de réduire la recroissance microbienne, en particulier dans des conditions occlusives.